(19) Europäisches Patentamt
European Patent Office
Office européen des brevets

(11) EP 3 400 964 A1

(12) **EUROPEAN PATENT APPLICATION**
published in accordance with Art. 153(4) EPC

(43) Date of publication:
**14.11.2018 Bulletin 2018/46**

(21) Application number: **17736173.0**

(22) Date of filing: **09.01.2017**

(51) Int Cl.:
***A61K 47/30** (2006.01)* ***A61K 9/00** (2006.01)*
***A61K 31/498** (2006.01)* ***A61K 31/4985** (2006.01)*

(86) International application number:
**PCT/KR2017/000290**

(87) International publication number:
**WO 2017/119801 (13.07.2017 Gazette 2017/28)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.01.2016 KR 20160002797**

(71) Applicant: **CTC Bio, Inc.**
**Hongcheon-gun, Gangwon-do 25142 (KR)**

(72) Inventors:
- **JEON, Hong Ryeol**
  **Suwon-si**
  **Gyeonggi-do 16543 (KR)**
- **LEE, Bong-Sang**
  **Suwon-si**
  **Gyeonggi-do 16501 (KR)**
- **PARK, Su-Jun**
  **Yongin-si**
  **Gyeonggi-do 16823 (KR)**
- **HAN, Jiyeong**
  **Ulsan 44003 (KR)**
- **KIL, Myeongcheol**
  **Iksan-si**
  **Jeollabuk-do 54597 (KR)**
- **KIM, Min Seop**
  **Seoul 02512 (KR)**
- **MEANG, Seul Ki**
  **Seoul 08249 (KR)**

(74) Representative: **Graf von Stosch**
**Patentanwaltsgesellschaft mbH**
**Prinzregentenstraße 22**
**80538 München (DE)**

# (54) TASTE-MASKED AND ORALLY ADMINISTERED PHARMACEUTICAL PREPARATION CONTAINING VARENICLINE OR PHARMACEUTICALLY ACCEPTABLE SALT THEREOF

(57) The present disclosure relates to a pharmaceutical preparation having varenicline or a pharmaceutically acceptable salt thereof as an active ingredient. More particularly, it relates to an orally administered pharmaceutical preparation which allows limited use of a sweetening agent or a flavoring agent by effectively masking the unique taste of varenicline, particularly bitterness, which is a bitter and burning taste, and ensures convenience in taking the pharmaceutical preparation by being orally administrable without irritation, while containing a pharmaceutically effective amount of varenicline or a pharmaceutically acceptable salt thereof, and a method for preparing the same.

EP 3 400 964 A1

## Description

### TECHNICAL FIELD

The present application claims priority to Korean Patent Application No. 10-2016-0002797 filed on January 8, 2016 in the Republic of Korea, the disclosures of which are incorporated herein by reference.

The present disclosure relates to a pharmaceutical preparation having varenicline or a pharmaceutically acceptable salt thereof as an active ingredient. More particularly, it relates to an orally administered pharmaceutical preparation which allows limited use of a sweetening agent or a flavoring agent by effectively masking the unique taste of varenicline, particularly bitterness, which is a bitter and burning taste, and ensures convenience in taking the pharmaceutical preparation by being orally administrable without irritation, while containing a pharmaceutically effective amount of varenicline or a pharmaceutically acceptable salt thereof, and a method for preparing the same.

### BACKGROUND ART

Varenicline has the following chemical formula:

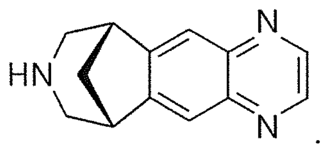

Varenicline is also called 5,8,14-triazatetracyclo[10.3.1.$0^{2,11}$.$0^{4,9}$]-hexadeca-2(11),3,5,7,9-pentane or 7,8,9,10-tetrahydro-6,10-methano-6H-pyrazino[2,3-h][3]-benzazepine. Varenicline and a pharmaceutically acceptable acid addition salt thereof are mentioned in International Patent Publication No. WO1999/35131.

Varenicline binds to the neuronal nicotinic acetylcholine receptor and is useful in regulating cholinergic action. Accordingly, varenicline is useful in treating various conditions or diseases, for example, inflammatory bowel disease (non-limiting examples: ulcerative colitis, pyoderma gangrenosum and Crohn's disease), irritable bowel syndrome, spasmodic dystonia, chronic pain, acute pain, celiac sprue, pouchitis, vasoconstriction, anxiety, panic disorder, depression, bipolar disorder, autism, sleep disorder, jet lag syndrome, amyotrophic lateral sclerosis (ALS), cognitive impairment, hypertension, bulimia, anorexia, obesity, cardiac arrhythmia, gastric acid hypersecretion, ulcer, pheochromocytoma, progressive supranuclear palsy, drug dependence and addiction (dependence on or addiction to, e.g., nicotine (and (or) tobacco products), alcohol, benzodiazepine, barbiturate, opioid or cocaine), headache, migraine, spasm, traumatic brain injury (TBI), obsessive-compulsive disorder (OCD), psychosis, Huntington's chorea, tardive dyskinesia, hyperkinesia, alexia, schizophrenia, multi-infarct dementia, age-related cognitive decline, epilepsy (including absence epilepsy), Alzheimer's disease (AD), Parkinson's disease (PD), attention deficit hyperactivity disorder (ADHD) and Tourette syndrome.

Varenicline tartrate is sold as a 1-mg or 0.5-mg tablet and is used as a smoking cessation aid to treat nicotine addiction or reduce tobacco addiction or consumption.

Varenicline can be administered through various routes. When considering the convenience of carrying and medication, it may be provided specifically as an oral formulation, more specifically an intraoral dispersible formulation. However, varenicline has a unique bitterness, i.e., a bitter and burning taste, and may cause an unpleasant sensation when swallowing. The bitterness problem remains unsolved even when varenicline is prepared into a salt (e.g., varenicline tartrate, varenicline salicylate, varenicline sulfate, varenicline fumarate, varenicline oxalate, varenicline hydrochloride, varenicline hydrobromide, varenicline citrate, varenicline maleate, varenicline succinate, varenicline phosphate, varenicline tosylate, varenicline free base and varenicline mesylate). Therefore, it is not easy to be prepared into a formulation for oral administration, particularly an intraoral dispersible formulation whose taste is directly felt during oral administration, e.g., an orally dissolving film, an orally disintegrating tablet, a suspension, a suspension tablet, a rapidly disintegrating tablet, an orally disintegrating capsule, an orally disintegrating granule, an orally disintegrating troche, a sublingual tablet, a powder and/or a chewable tablet. Especially, it is difficult to be used as an active ingredient of an orally administered pharmaceutical preparation for treating a condition or a disease requiring regular medication for a long period of time.

There have been attempts to mask the bitterness of varenicline or a pharmaceutically acceptable salt thereof using various ingredients known to have taste masking (TM) effect in the art, such as a sweetening agent, a flavoring agent, etc. However, these ingredients cannot solve the irritation problem during swallowing. Accordingly, development of an orally administered pharmaceutical preparation with the bitterness of varenicline or a pharmaceutically acceptable salt thereof masked is necessary.

## DISCLOSURE

### Technical Problem

The present disclosure is directed to providing an orally administered pharmaceutical preparation containing varenicline as an active ingredient, which can be taken regularly for a long period of time by effectively masking the unique bitterness of varenicline and removing irritation during swallowing, thereby effectively masking unpleasant sensation when the pharmaceutical preparation is administered orally, and a method for preparing the same.

### Technical Solution

In order to solve the technical problems described above, the present disclosure provides an orally administered pharmaceutical preparation containing varenicline or a pharmaceutically acceptable salt thereof as an active ingredient and containing both an anionic polymer containing carboxyl groups and a cationic polymer containing amino groups as a bitter taste masking agent, a method for preparing the same and a method for treating a subject in need thereof by orally administering the same.

The inventors of the present disclosure have identified that, although ion-exchange resins have been used to mask the bitterness of drugs prior to this application, it is difficult to effectively the strong bitterness of varenicline or a salt thereof only with the ion-exchange resins. They have found out that use of an anionic polymer containing carboxyl groups and a cationic polymer containing amino groups in combination is very effective in masking the unique bitterness of varenicline or a pharmaceutically acceptable salt even with a small amount due to their synergy effect, allows the masking of bitterness without using another sweetening agent or flavoring agent and can reduce irritation during swallowing and have completed the present disclosure.

In an aspect, the present disclosure provides a bitterness-masked orally administered pharmaceutical preparation containing varenicline or a pharmaceutically acceptable salt thereof as an active ingredient and containing both an anionic polymer containing carboxyl groups and a cationic polymer containing amino groups.

In the present disclosure, varenicline or a pharmaceutically acceptable salt thereof is used as an active ingredient.

In the present disclosure, the 'active ingredient' means a therapeutically active compound, any prodrug thereof or a pharmaceutically acceptable salt or solvate of the compound or the prodrug.

In the present disclosure, the 'varenicline' includes a parent drug, a prodrug thereof and a pharmaceutically acceptable salt or solvate of the parent drug or the prodrug. The parent drug of varenicline is described in WO99/35131, the disclosures of which are incorporated herein by reference. A method for preparing varenicline is described in US Patent No. 6,410,550, the disclosures of which are incorporated herein by reference. The resolution of a racemic mixture of varenicline is described in WO01/62736, the disclosures of which are incorporated herein by reference.

The pharmaceutical preparation according to the present disclosure may contain, instead of or together with varenicline, a varenicline derivative as an active ingredient. Accordingly, the pharmaceutical preparation according to the present disclosure may contain a derivative having pharmacological activity comparable to that of varenicline.

In the present disclosure, 'pharmaceutically acceptable' means chemical, physical and/or toxicological compatibility with other ingredients of a composition and/or a mammal treated with the same.

In the present disclosure, the 'pharmaceutically acceptable salt' refers to a nontoxic acid addition salt derived from an inorganic acid or an organic acid. Adequate salt derivatives include, for example, halide, tosylate, mesylate, thiocyanate, sulfate, bisulfate, sulfite, bisulfite, aryl sulfonate, alkyl sulfate, fumarate, oxalate, phosphonate, phosphate, monohydrogen phosphate, dihydrogen phosphate, metaphosphate, pyrophosphate, alkanoate, cycloalkyl alkanoate, aryl alkanoate, adipate, alginate, aspartate, benzoate, glucoheptanoate, glycerophosphate, lactate, maleate, nicotinate, palmitate, pectinate, picrate, pivalate, succinate, tartrate, citrate, camphorate, camphorsulfonate, digluconate, trifluoroacetate, hydrochloride, hydrobromide, salicylate, etc. and are not limited as long as they are pharmaceutically acceptable salts having the unique bitterness of varenicline. Specifically, the varenicline or a pharmaceutically acceptable salt thereof may be one or more selected from a group consisting of varenicline salicylate, varenicline sulfate, varenicline fumarate, varenicline oxalate, varenicline hydrochloride, varenicline hydrobromide, varenicline citrate, varenicline maleate, varenicline succinate, varenicline phosphate, varenicline tosylate and varenicline mesylate. The acid addition salt may be prepared by a common method. For example, it may be prepared by dissolving the compound in an excess amount of aqueous acid solution and precipitating the salt using a water-miscible organic solvent, e.g., methanol, ethanol, acetone or acetonitrile. After heating the compound and an acid in water or an alcohol, the salt precipitated by evaporating and drying the mixture may be filtered through suction.

The active ingredient according to the present disclosure may be varenicline or a pharmaceutically acceptable salt thereof without limitation. Specifically, varenicline salicylate, varenicline sulfate, varenicline fumarate, varenicline oxalate, varenicline hydrochloride, varenicline hydrobromide, varenicline citrate, varenicline maleate, varenicline succinate, varenicline phosphate, varenicline tosylate or varenicline mesylate may be preferred to varenicline tartrate. More specifically, varenicline sulfate, varenicline hydrochloride, varenicline salicylate, varenicline hydrobromide or varenicline free base may be used. Most specifically, varenicline salicylate may be used. Varenicline salicylate is superior in terms of stability against oxidation.

In the present disclosure, both the anionic polymer containing carboxyl groups and the cationic polymer containing amino groups are used as bitter taste masking agents.

In the present disclosure, the 'ingredient' refers to any substance that constitutes a pharmaceutical preparation with varenicline, a prodrug, a pharmaceutically acceptable salt or a solvate thereof, such as an excipient, a plasticizer, a disintegrant, a diluent, a solvent, a penetration enhancer, a preservative, a buffer, a gelator, a lubricant, a carrier, a stabilizer, a gel, a dye, a pigment, a surfactant, an inert filler, an adhesive, a texturizer, a softener, an emulsifier and a mixture thereof.

In the present disclosure, as the 'anionic polymer containing carboxyl groups', any one which is nontoxic and does not negatively affect the pharmacological activity of varenicline or a pharmaceutically acceptable salt thereof may be used without limitation. Specifically, polyacryls may be used. More specifically, a crosslinked polymer of acrylic acid and divinylbenzene, a crosslinked polymer of methacrylic acid and divinylbenzene (e.g., Kyron-T-104®, Kyron-T-114®, Kyron-T-134® AmberliteIRP64®, AmberliteIRP88®, AmberliteIRC50®, TulsionT339®, TulsionT 335®, Purolite C102D®, PuroliteC108DR®, PuroliteC115HMR®, PuroliteC115KMR®, INDION204®, INDION214®, INDION234®, INDION234S®, INDION264®, INDION414®, INDION464®, INDION294®, etc., although not being limited thereto) or a copolymer of methacrylic acid and methyl methacrylate (e.g., Eudragit®L30 D-55, Eudragit® L 100-55, Eudragit® L 100, Eudragit® L 12,5, Eudragit® S 100, Eudragit® S12,5, Eudragit® FS 30D, etc., although not being limited thereto) or the acrylic acid copolymer Carbomer may be used. It is ionized in the form of $COO^-$ in an aqueous solution and exists in the form of $COO^-Na^+$, $COO^-K^+$ or COOH in a solid phase.

The pharmaceutical preparation composition according to the present disclosure may contain the anionic polymer containing carboxyl groups in an amount of 1-60 wt%, more specifically 3-20 wt%, based on the total weight of the composition. If the amount is less than 1 wt%, it is difficult to achieve a bitterness masking effect. And, if it exceeds 60 wt%, the oral disintegration rate, texture and convenience of medication worsen significantly because the total weight of the pharmaceutical preparation is increased and portability may worsen due to increased size.

In the present disclosure, as the 'cationic polymer containing amino groups', any natural cationic polymer or synthetic cationic polymer which is nontoxic and does not negatively affect the pharmacological activity of varenicline or a pharmaceutically acceptable salt thereof may be used without limitation. Specifically, a cationic polymer containing amino groups polymerized from acrylic acid, methacrylic acid or styrene as a monomer may be used. For example, aminoalkyl methacrylate copolymers, which are cationic methacrylic acid copolymers, include EudragitE100®, EudragitEPO® and EudragitE12.5®, styrene-based copolymers include KyronT-123®, Indion454® and acrylic acid copolymers include Purolite A830EMR®, etc. In addition, natural cationic polymers include lecithin, chitosan, gelatin, etc., although not being limited thereto.

The pharmaceutical preparation composition according to the present disclosure may contain the cationic polymer, specifically the cationic polymer containing amino groups, in an amount of 1-60 wt%, more specifically 2-20 wt%, based on the total weight of the composition. If the amount is less than 1 wt%, it is difficult to achieve a bitterness masking effect. And, if it exceeds 60 wt%, the oral disintegration rate, texture and convenience of medication worsen significantly because the total weight of the pharmaceutical preparation is increased and portability may worsen due to increased size.

In the pharmaceutical preparation according to the present disclosure, the weight ratio of the total weight of the anionic polymer containing carboxyl groups based on the total weight of the varenicline or a pharmaceutically acceptable salt thereof may be specifically 10:1 to 1:100, more specifically 10:1 to 1:10. If the weight ratio is less than 10:1, it is difficult to achieve a bitterness masking effect. And, if it exceeds 1:100, the oral disintegration rate, texture and convenience of medication worsen significantly because the total weight of the pharmaceutical preparation is increased and portability may worsen due to increased size.

In the pharmaceutical preparation according to the present disclosure, the weight ratio of the total weight of the cationic polymer based on the total weight of the varenicline or a pharmaceutically acceptable salt thereof may be specifically 10:1 to 1:100, more specifically 10:1 to 1:10. If the weight ratio is less than 10:1, it is difficult to achieve a bitterness masking effect. And, if it exceeds 1:100, the oral disintegration rate, texture and convenience of medication worsen significantly because the total weight of the pharmaceutical preparation is increased and portability may worsen due to increased size.

In the pharmaceutical preparation according to the present disclosure, the weight ratio of the total weight of the anionic polymer containing carboxyl groups and the cationic polymer containing amino groups based on the total weight of the varenicline or a pharmaceutically acceptable salt thereof may be specifically 10:1 to 1:100, more specifically 10:1 to 1:10. If the weight ratio is less than 10:1, it is difficult to achieve a bitterness masking effect. And, if it exceeds 1:100, the oral disintegration rate, texture and convenience of medication worsen significantly because the total weight

of the pharmaceutical preparation is increased and portability may worsen due to increased size.

In the pharmaceutical preparation according to the present disclosure, the anionic polymer containing carboxyl groups and the cationic polymer containing amino groups synergistically exert a superior bitterness masking effect. The bitterness masking effect is maximized when the weight ratio of the total weight of the cationic polymer containing amino groups based on the total weight of the anionic polymer containing carboxyl groups is 10:1 to 1:100, more specifically 5:1 to 1:4. If the weight ratio of the total weight of the cationic polymer containing amino groups based on the total weight of the anionic polymer containing carboxyl groups is less than 10:1, it is difficult to achieve a bitterness masking effect. And, if it exceeds 1:100, the oral disintegration rate, texture and convenience of medication worsen significantly because the total weight of the pharmaceutical preparation is increased and portability may worsen due to increased size.

In the pharmaceutical preparation according to the present disclosure, the weight ratio of the total weight of the anionic polymer containing carboxyl groups and the cationic polymer containing amino groups based on the total weight of the varenicline or a pharmaceutically acceptable salt thereof may be 10:1 to 1:100 (varenicline or pharmaceutically acceptable salt thereof : anionic polymer containing carboxyl groups and cationic polymer containing amino groups), more specifically 10:1 to 1:10. If the weight ratio is less than 10:1, it is difficult to achieve a bitterness masking effect. And, if it exceeds 1:100, the oral disintegration rate, texture and convenience of medication worsen significantly because the total weight of the pharmaceutical preparation is increased and portability may worsen due to increased size.

In the pharmaceutical preparation according to the present disclosure, the weight ratio of the total weight of the varenicline or a pharmaceutically acceptable salt thereof, the weight ratio of the anionic polymer containing carboxyl groups and the weight ratio of the cationic polymer containing amino groups may be 10:1:1 to 1:100:100 (varenicline or pharmaceutically acceptable salt thereof : anionic polymer containing carboxyl groups : cationic polymer containing amino groups), more specifically 10:1:1 to 1:10:10 (varenicline or pharmaceutically acceptable salt thereof : anionic polymer, specifically anionic polymer containing carboxyl groups : cationic polymer, specifically cationic polymer containing amino groups), more specifically 5:4:1 to 1:100:100, further more specifically 1:8:2 to 1:40:10, most specifically 1:8:2. If the weight ratio is less than 10:1:1, it is difficult to achieve a bitterness masking effect. And, if it exceeds 1:100:100, the oral disintegration rate, texture and convenience of medication worsen significantly because the total weight of the pharmaceutical preparation is increased and portability may worsen due to increased size.

The pharmaceutical preparation according to the present disclosure may be prepared as an orally administered formulation. For example, it may be prepared into various formulations, e.g., a tablet, a film, a suspension, a granule, a gel, a pill, a tincture, a decoction, an infusion, a spirit, a fluid extract, an elixir, an extract, a syrup, a powder, an aromatic water, a lemonade, etc. And, the tablet may be prepared into various forms, e.g., an orally disintegrating tablet, a mucoadhesive tablet, a dispersible tablet, a sublingual tablet, a buccal tablet, a chewable tablet, an effervescent tablet, a solution tablet, etc. In addition, those of ordinary skill can make various modifications to the tablet as desired. More specifically, it may be an intraoral dispersible formulation, e.g., an orally dissolving film, an orally disintegrating tablet, a suspension, a suspension tablet, a rapidly disintegrating tablet, an orally disintegrating granule, an orally disintegrating troche, a sublingual tablet, a powder and/or a chewable tablet, which is dissolved, dispersed or disintegrated in liquids or in the mouth. When considering the situation where the pharmaceutical preparation is administered, portability and various purposes, the pharmaceutical preparation according to the present disclosure may be prepared as a suspension formulation, an orally dissolving film formulation, a rapidly disintegrating tablet or an orally disintegrating granule. Specifically, 80% or more of the formulations may be dissolved, dispersed or disintegrated within 10 minutes or 5 minutes after oral administration. The term orally dissolving film may be used interchangeably with a film, a strip, an orally disintegrating film, etc. and refers to a formulation which is adhered in the oral cavity, e.g., on the tongue, on the oral mucosa, below the tongue, etc. The orally disintegrating film or rapidly disintegrating tablet according to the present disclosure is advantageous in that it can be administered without water.

When the orally administered pharmaceutical preparation according to the present disclosure is prepared as an orally dissolving film, a polymer has to be contained to form the film. Because the orally administered pharmaceutical preparation according to the present disclosure contains many anionic ingredients, compatibility with the polymer is important. Therefore, pullulan, hydroxypropyl cellulose (HPC), hydroxypropyl methylcellulose (HPMC), polyvinylpyrrolidone (PVP), starch, a polyethylene glycol-polyvinyl alcohol copolymer, copovidone, hydroxyethyl cellulose, hydroxypropyl starch, polyethylene oxide, poloxamer or a mixture thereof may be specifically used. The polymer may be contained in an amount of 20-50 wt% based on the weight of the dried film, although not being limited thereto.

The orally administered pharmaceutical preparation according to the present disclosure may further contain a pharmaceutically acceptable carrier that can be commonly added to a pharmaceutical preparation. The pharmaceutically acceptable carrier may contain an additive commonly used in the pharmaceutical field such as an excipient, a plasticizer, a disintegrant, a diluent, a solvent, a penetration enhancer, a preservative, a buffer, a gelator, a lubricant, a carrier, a stabilizer, a gel, a dye, a pigment, a surfactant, an inert filler, an adhesive, a texturizer, a softener, an emulsifier and a mixture thereof.

The orally administered pharmaceutical preparation according to the present disclosure may contain 40-99.7 wt% of the additive based on the total weight of the preparation. When the pharmaceutical preparation is an orally

disintegrating film, it may contain 90-99.8 wt% of the additive based on the total weight of the preparation. When the pharmaceutical preparation is a rapidly disintegrating tablet, it may contain 90-99.8 wt% of the additive based on the total weight of the preparation.

A suitable binder or excipient includes, for example, cellulose, methyl cellulose, ethyl cellulose, hydroxypropyl cellulose, hydroxymethyl cellulose, polypropylpyrrolidone, polyvinylpyrrolidone, gelatin, polyethylene glycol, starch, natural or synthetic gum (e.g., alginate or gum arabic), mannitol, microcrystalline cellulose, anhydrous calcium hydrogen phosphate, sorbitol, L-HPC (low-substituted hydroxypropyl cellulose), pregelatinized starch, lactose or a mixture thereof, although not being limited thereto. Specifically, the binder or excipient may be contained in an amount of 0.01-90 wt% based on the total weight of the orally administered pharmaceutical preparation. When the pharmaceutical preparation is an orally disintegrating film, it may contain 1-90 wt% of the binder or excipient based on the total weight of the pharmaceutical preparation. When the pharmaceutical preparation is a rapidly disintegrating tablet, it may contain 1-99.8 wt% of the binder or excipient based on the total weight of the pharmaceutical preparation.

A suitable lubricant includes, for example, calcium stearate, glyceryl monostearate, glyceryl palmitostearate, magnesium stearate, polyethylene glycol, sodium benzoate, sodium lauryl sulfate, sodium stearyl fumarate, stearic acid, talc and zinc stearate, although not being limited thereto. Specifically, the lubricant may be contained in an amount of 0.1-3 wt% based on the total weight of the orally administered pharmaceutical preparation. When the pharmaceutical preparation is a rapidly disintegrating tablet, it may contain 0.1-5 wt% of the lubricant based on the total weight of the pharmaceutical preparation.

A suitable disintegrant includes, for example, sodium starch glycolate, sodium carboxymethyl cellulose, calcium carboxymethyl cellulose, croscarmelose sodium, polyvinylpyrrolidone, methyl cellulose, microcrystalline cellulose, powdered cellulose, lower alkyl-substituted hydroxypropyl cellulose, polacrilin potassium, starch, pregelatinized starch, sodium alginate, etc., although not being limited thereto. Specifically, the disintegrant may be contained in an amount of 1-80 wt% based on the total weight of the orally administered pharmaceutical preparation. When the pharmaceutical preparation is an orally disintegrating film, it may contain 3-45 wt% of the disintegrant based on the total weight of the pharmaceutical preparation. When the pharmaceutical preparation is a rapidly disintegrating tablet, it may contain 0.1-90 wt% of the disintegrant based on the total weight of the pharmaceutical preparation.

If necessary, a sweetener, a flavor and/or a colorant may be further contained, but the sweetener, flavor and/or colorant may cause irritation during swallowing. The sweetener, flavor and/or colorant may be contained in an amount of 5 wt% or less, more specifically 3 wt% or less, most specifically 1 wt% or less, based on the total weight of the orally administered pharmaceutical preparation according to the present disclosure.

When the pharmaceutical preparation of the present disclosure is used to treat dependence on or addiction to nicotine or tobacco products or as a smoking cessation aid, it may further contain, in addition to the varenicline or the pharmaceutically acceptable salt thereof, another drug as an active ingredient as long as the purpose of the present disclosure is not negatively affected. For example, it may contain one or more selected from a group consisting of an $\alpha$7-nicotinic acetylcholine receptor antagonist, an agent for treating withdrawal symptoms (e.g., bupropion, rimonabant, dihydroerysodine, dopamine, mecamylamine, cytisine, 3-methylaminoisocamphane, baclofen, clopazolin, butanone, etc.), a nicotine-specific antibody, an anti-protein antibody, a natural product (e.g., angelicae radix, zizyphus seed, red ginseng, skullcap, etc.) or a combination thereof. Those of ordinary skill can select various drugs in addition to the above-described drugs, if necessary.

The pharmaceutical preparation according to the present disclosure may be used for any disease, disorder and/or symptom treated by administering the varenicline or the pharmaceutically acceptable salt thereof as an active ingredient without limitation. For example, it may be used for a disorder or disease including inflammatory bowel disease, ulcerative colitis, pyoderma gangrenosum, Crohn's disease, irritable bowel syndrome, spasmodic dystonia, chronic pain, acute pain, celiac sprue, pouchitis, vasoconstriction, anxiety, panic disorder, depression, bipolar disorder, autism, sleep disorder, jet lag syndrome, amyotrophic lateral sclerosis (ALS), cognitive impairment, hypertension, bulimia, anorexia, obesity, cardiac arrhythmia, gastric acid hypersecretion, ulcer, pheochromocytoma, progressive supranuclear palsy, drug dependence and addiction (dependence on or addiction to, e.g., nicotine, tobacco products, alcohol, benzodiazepine, barbiturate, opioid or cocaine), headache, apoplexy, traumatic brain injury (TBI), obsessive-compulsive disorder (OCD), psychosis, Huntington's chorea, tardive dyskinesia, hyperkinesia, alexia, schizophrenia, multi-infarct dementia, age-related cognitive decline, epilepsy (including absence epilepsy), Alzheimer's disease (AD), Parkinson's disease (PD), attention deficit hyperactivity disorder (ADHD), Tourette syndrome and other similar disorders or diseases known in the art (although not being limited thereto). Specifically, it may be used to treat dependence on or addiction to nicotine or tobacco products.

In the present disclosure, 'treatment' means any action of improving or beneficially altering a (acute or chronic) disease, a disorder or a symptom resulting therefrom by administering the pharmaceutical preparation. In a broad sense, the 'treatment' includes 'prevention', which means any action of preventing the occurrence of a disease or a symptom resulting therefrom or delaying the occurrence by administering the pharmaceutical preparation. The 'treatment' includes, for example, the interruption, alleviation, improvement, stopping, suppression, delaying, reversal, etc. of a (acute or

chronic) disease, a disorder or a symptom resulting therefrom.

The dosage, frequency and continuance of administration will vary depending on such factors as the characteristics and seriousness of the condition to be treated, the age and general physical conditions of a subject (host) and the resistance of the subject (host) to the active ingredient. The pharmaceutical preparation may be provided as a once-daily administration form, a multiple-daily administration form or a once-weekly administration form. The prescription may last from about 2-3 days to several weeks or longer.

In an exemplary embodiment, the pharmaceutical preparation according to the present disclosure may contain 0.5-1 mg of the varenicline or the pharmaceutically acceptable salt thereof for a single-dose unit. More specifically, although the dosage of the varenicline or a pharmaceutically acceptable salt thereof contained in a single-dose unit of the pharmaceutical preparation may vary depending on the specific medicinal use, the unique taste of the varenicline or a pharmaceutically acceptable salt thereof is effectively masked when the anionic polymer containing carboxyl groups and the cationic polymer as the taste masking ingredients are added in combination to 0.5-1 mg of the varenicline or the pharmaceutically acceptable salt thereof for a single-dose unit. In a specific exemplary embodiment, because the varenicline or a pharmaceutically acceptable salt thereof corresponding to a daily dosage of the single-dose unit can be taken without discomfort, the daily dosage of the single-dose unit can be taken at once. Accordingly, a continuous medication regimen of taking the drug every day can be changed into a periodic medication regimen of taking the drug with predetermined intervals. Through this, the side effects that may occur in the continuous medication regimen can be resolved, although the present disclosure is not limited to such effect.

In an exemplary embodiment, the present disclosure may provide a single-dose unit of an orally administered pharmaceutical preparation, which contains: 0.5-1 mg of the varenicline or a pharmaceutically acceptable salt thereof; and 0.05-100 mg of the anionic polymer containing carboxyl groups and 0.05-100 mg of the cationic polymer.

In another aspect, the present disclosure provides a method for preparing a bitterness-masked orally administered pharmaceutical preparation, which contains varenicline or a pharmaceutically acceptable salt thereof as an active ingredient and contains both an anionic polymer containing carboxyl groups and a cationic polymer containing amino groups.

The present disclosure provides a method for masking the bitterness of an orally administered pharmaceutical preparation containing varenicline or a pharmaceutically acceptable salt thereof as an active ingredient by adding an anionic polymer containing carboxyl groups and a cationic polymer containing amino groups together.

When the anionic polymer containing carboxyl groups and the cationic polymer containing amino groups are added together, the bitterness of the orally administered pharmaceutical preparation containing varenicline or a pharmaceutically acceptable salt thereof as an active ingredient can be masked.

In a specific exemplary embodiment, the orally administered pharmaceutical preparation may be prepared by adding the anionic polymer containing carboxyl groups and the cationic polymer containing amino groups to the varenicline or a pharmaceutically acceptable salt thereof in liquid state and dissolving the same through stirring. Through this, the orally administered pharmaceutical preparation containing the varenicline or a pharmaceutically acceptable salt thereof as an active ingredient which can be administered without water may be prepared.

In another aspect, the present disclosure provides a method for administering an orally administered pharmaceutical preparation containing varenicline or a pharmaceutically acceptable salt thereof as an active ingredient and containing both an anionic polymer containing carboxyl groups and a cationic polymer containing amino groups with bitterness masked by administering the orally administered pharmaceutical preparation to a patient in need thereof.

## Advantageous Effects

According to the present disclosure, the unique bitter taste of varenicline is effectively masked and the irritation during swallowing is removed. Accordingly, the unpleasantness when orally administering a pharmaceutical preparation containing varenicline as an active ingredient is effectively masked and the pharmaceutical preparation can be administered orally for a long period of time.

## BEST MODE

Hereinafter, the present disclosure will be described in detail with reference to the following examples. However, it should be understood that the following examples are not intended to limit the scope of the present disclosure and various changes and modifications may be made without departing from the spirit and scope of the invention as defined in the following claims. It will be apparent that these exemplary embodiments are provided so that the present disclosure will be complete and understood easily by those skilled in the art.

[Example 1. Preparation of film formulation containing varenicline tartrate as active ingredient]

After adding an anionic polymer containing carboxyl groups and a cationic polymer containing amino groups to varenicline tartrate dissolved in a solution, the mixture was stirred for at least 1 hour.

Then, after adding a plasticizer, a flavoring agent, a coloring agent, a sweetening agent, a surfactant and an excipient (diluent) to purified water and dissolving or dispersing by stirring, the mixture was homogenized using a homogenizer (Ultra-Turrax T-25, IKA). Then, after adding a polymer (pullulan) and then homogenizing using the same homogenizer, a gas was removed from the solution for preparing a film in vacuo and the solution was coated on a PET film to an appropriate thickness. Then, a film formulation containing varenicline tartrate was prepared by drying the same at 60-80 °C.

[Example 2. Preparation of film formulation containing varenicline salicylate as active ingredient]

After adding an anionic polymer containing carboxyl groups and a cationic polymer containing amino groups to varenicline salicylate dissolved in a solution, the mixture was stirred for at least 1 hour.

Then, after adding a plasticizer, a flavoring agent, a coloring agent, a sweetening agent, a surfactant and an excipient (diluent) to purified water and dissolving or dispersing by stirring, the mixture was homogenized using a homogenizer (Ultra-Turrax T-25, IKA). Then, after adding a polymer (pullulan) and then homogenizing using the same homogenizer, a gas was removed from the solution for preparing a film in vacuo and the solution was coated on a PET film to an appropriate thickness. Then, a film formulation containing varenicline salicylate was prepared by drying the same at 60-80 °C.

[Example 3. Preparation of rapidly disintegrating tablet containing varenicline tartrate as active ingredient]

After adding an anionic polymer containing carboxyl groups and a cationic polymer containing amino groups to varenicline tartrate dissolved in a solution, the mixture was stirred for at least 1 hour.

Then, a solid was obtained by filtering and drying the solution. After adding a binder, a disintegrant, an excipient (diluent) and a lubricant to the obtained solid and then mixing the same, a tablet was prepared using a tablet-making machine.

[Example 4. Preparation of rapidly disintegrating tablet containing varenicline salicylate as active ingredient]

After adding an anionic polymer containing carboxyl groups and a cationic polymer containing amino groups to varenicline salicylate dissolved in a solution, the mixture was stirred for at least 1 hour.

Then, a solid was obtained by filtering and drying the solution. After adding a binder, a disintegrant, an excipient (diluent) and a lubricant to the obtained solid and then mixing the same, a tablet was prepared using a tablet-making machine.

[Example 5. Preparation of granule containing varenicline tartrate as active ingredient]

After adding an anionic polymer containing carboxyl groups and a cationic polymer containing amino groups to varenicline tartrate dissolved in a solution, the mixture was stirred for at least 1 hour.

Then, a solid was obtained by filtering and drying the solution. Separately from this, a solution containing a binder, a disintegrant and an excipient (diluent) was prepared and the obtained solid was added into a fluidized-bed granulator and then spray-dried to prepare a granule.

[Example 6. Preparation of granule containing varenicline salicylate as active ingredient]

After adding an anionic polymer containing carboxyl groups and a cationic polymer containing amino groups to varenicline salicylate dissolved in a solution, the mixture was stirred for at least 1 hour.

Then, a solid was obtained by filtering and drying the solution. Separately from this, a solution containing a binder, a disintegrant and an excipient (diluent) was prepared and the obtained solid was added into a fluidized-bed granulator and then spray-dried to prepare a granule.

[Example 7. Preparation of suspension containing varenicline tartrate as active ingredient]

After adding an anionic polymer containing carboxyl groups and a cationic polymer containing amino groups to varenicline tartrate dissolved in a solution, the mixture was stirred for at least 1 hour. Then, after adding a flavoring agent, a coloring agent, a sweetening agent, a surfactant and a viscosity increasing agent to purified water, a suspension was prepared by stirring the mixture.

[Example 8. Preparation of suspension containing varenicline salicylate as active

ingredient]

After adding an anionic polymer containing carboxyl groups and a cationic polymer containing amino groups to varenicline salicylate dissolved in a solution, the mixture was stirred for at least 1 hour. Then, after adding a flavoring agent, a coloring agent, a sweetening agent, a surfactant and a viscosity increasing agent to purified water, a suspension was prepared by stirring the mixture.

[Test Example 1. Test of bitterness masking effect of film formulation containing varenicline tartrate as active ingredient when anionic polymer containing carboxyl groups and cationic polymer containing amino groups are used]

A film formulation containing varenicline tartrate as an active ingredient was prepared in the same manner as in Example 1, using an anionic polymer containing carboxyl groups alone, a cationic polymer containing amino groups alone or a combination of an anionic polymer containing carboxyl groups and a cationic polymer containing amino groups as described in Table 1.

The bitterness masking effect of the film formulation was tested as follows. The TM sensory test was performed for the formulations of the same weight. A test subject put the formulation sample containing varenicline tartrate of the same amount in the mouth, dissolved it for the same time period, spitted it out and then lightly rinsed the mouth with the same amount of water. Then, the time for which bitter and burning taste was maintained was recorded. The time between tests for each formulation sample was set to 3 hours or longer and the subject who felt the bitter and burning taste longer than 3 hours was excluded from the next test.

The result is shown in Table 1.

[Table 1]

| | | Comparative Example | Example (%) | | | | | |
|---|---|---|---|---|---|---|---|---|
| | | 1 | 2 | 3 | 4 | 5 | 6 | 7 |
| Varenicline Tartrate | | 0.24 | | | | | | |
| Taste masking agent | (anionic polymer) Purolite C108DR | - | 2.33 | - | 2.31 | 2.31 | - | - |
| | (anionic polymer) Eudragit L100 | - | - | 2.33 | - | - | 2.31 | 2.31 |
| | (cationic polymer) Purolite A830EMR | - | - | - | 0.93 | - | 0.93 | - |
| | (cationic polymer) Eudragit EPO | - | - | - | - | 0.930 | - | 0.93 |
| Pullulan | | 13.0 | | | | | | |
| Plasticizer | | 7.0 | | | | | | |
| Aspartame | | 3.0 | | | | | | |
| Pigment | | 10.0 | | | | | | |
| Diluent | | Q.S | | | | | | |
| Flavor | | Q.S | | | | | | |
| Water | | to 100% | | | | | | |

(continued)

| | Comparative Example | Example (%) | | | | | |
|---|---|---|---|---|---|---|---|
| | 1 | 2 | 3 | 4 | 5 | 6 | 7 |
| Taste masking value | 1 | 2 | 2 | 4 | 4 | 3 | 3 |

* Taste masking value

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |
| (NLT: no less than, NMT: no more than) | |

As seen from Table 1, the bitterness masking effect was remarkably superior when the anionic polymer containing carboxyl groups and the cationic polymer containing amino groups were used together as compared to when the anionic polymer containing carboxyl groups was used alone.

[Test Example 2. Test of bitterness masking effect of film formulation containing varenicline tartrate as active ingredient depending on weight ratio of anionic polymer containing carboxyl groups and cationic polymer containing amino groups]

A film formulation containing varenicline tartrate as an active ingredient was prepared in the same manner as in Example 1, using a combination of an anionic polymer containing carboxyl groups and a cationic polymer containing amino groups as described in Table 2 by varying their weight ratio.

The bitterness masking effect of the film formulation was tested in the same manner as described in Test Example 1.

The result is shown in Table 2.

[Table 2]

| | | Comparative Example | Example % | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| Ratio (API: TM agent) | | - | 10:1 | | | | | 1:10 | | | | | 1:100 | | | | |
| Ratio of TM agent (Anionic polymer: Cationic polymer) | | | 1:10 | 5:1 | 4:1 | 1:4 | 20:1 | 1:10 | 5:1 | 4:1 | 1:4 | 20:1 | 1:10 | 5:1 | 4:1 | 1:4 | 20:1 |
| Varenicline Tartrate | | 0.25 | 0.24 | | | | | 0.23 | | | | | 0.19 | | | | |
| Taste masking agent | Purolite C108DR | - | 0.002 | 0.019 | 0.019 | 0.005 | 0.019 | 0.210 | 1.941 | 1.867 | 0.467 | 2.220 | 1.751 | 16.049 | 15.426 | 3.857 | 18.359 |
| | Eudragit EPO | - | 0.021 | 0.004 | 0.005 | 0.019 | 0.001 | 2.100 | 0.387 | 0.467 | 1.867 | 0.107 | 17.513 | 3.202 | 3.857 | 15.426 | 0.918 |
| Pullulan | | 13.0 | | | | | | | | | | | | | | | |
| Plasticizer | | 7.0 | | | | | | | | | | | | | | | |
| Aspartame | | 3.0 | | | | | | | | | | | | | | | |
| Pigment | | 10.0 | | | | | | | | | | | | | | | |
| Diluent | | Q.S | | | | | | | | | | | | | | | |
| Flavor | | Q.S | | | | | | | | | | | | | | | |
| Water | | to 100% | | | | | | | | | | | | | | | |
| Effect of taste masking | | 1 | 2 | 3 | 3 | 3 | 1 | 3 | 4 | 4 | 4 | 2 | 3 | 4 | 4 | 4 | 3 |

* Taste masking value

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |
| (NLT: no less than, NMT: no more than) | |

As seen from Table 2, superior bitterness masking effect was achieved for the varenicline tartrate (API) when the weight ratio of the anionic polymer containing carboxyl groups and the cationic polymer containing amino groups used as the bitter taste masking agents was in the range from 5:1 to 1:4.

[Test Example 3. Test of bitterness masking effect of rapidly disintegrating tablet containing varenicline tartrate as active ingredient depending on weight ratio of anionic polymer containing carboxyl groups and cationic polymer containing amino groups]

A rapidly disintegrating tablet containing varenicline tartrate as an active ingredient was prepared in the same manner as in Example 1, using a combination of an anionic polymer containing carboxyl groups and a cationic polymer containing amino groups as described in Table 3 by varying their weight ratio.

The bitterness masking effect of the rapidly disintegrating tablet was tested in the same manner as described in Test Example 1.

The result is shown in Table 3.

[Table 3]

| | | Comparative Example | Example % | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| Ratio (API: TM agent) | | - | 10:1 | | | | | 1:10 | | | | | 1:100 | | | | |
| Ratio of TM agent (Anionic polymer: Cationic polymer) | | | 1:10 | 5:1 | 4:1 | 1:4 | 20:1 | 1:10 | 5:1 | 4:1 | 1:4 | 20:1 | 1:10 | 5:1 | 4:1 | 1:4 | 20:1 |
| Varenicline Tartrate | | 0.25 | 0.24 | | | | | 0.23 | | | | | 0.19 | | | | |
| Taste masking agent | Purolite C108DR | - | 0.002 | 0.019 | 0.019 | 0.005 | 0.019 | 0.210 | 1.941 | 1.867 | 0.467 | 2.220 | 1.751 | 16.049 | 15.426 | 3.857 | 18.359 |
| | Eudragit EPO | - | 0.021 | 0.004 | 0.005 | 0.019 | 0.001 | 2.100 | 0.387 | 0.467 | 1.867 | 0.107 | 17.513 | 3.202 | 3.857 | 15.426 | 0.918 |
| Binder & Disintegration agent & Diluent | | 60.0 | | | | | | | | | | | | | | | |
| Aspartame | | 7.0 | | | | | | | | | | | | | | | |
| Flavor (If needed) | | Q.S | | | | | | | | | | | | | | | |
| Lubricant (If needed) | | Q.S | | | | | | | | | | | | | | | |
| Water | | to 100% | | | | | | | | | | | | | | | |
| Effect of taste masking | | 1 | 2 | 3 | 3 | 3 | 1 | 3 | 4 | 4 | 4 | 3 | 3 | 4 | 4 | 4 | 3 |

* Taste masking value

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |
| (NLT: no less than, NMT: no more than) | |

As seen from Table 3, superior bitterness masking effect was achieved for the varenicline tartrate (API) when the weight ratio of the anionic polymer containing carboxyl groups and the cationic polymer containing amino groups used as the bitter taste masking agents was in the range from 5:1 to 1:4.

[Test Example 4. Test of film formulation depending on weight ratio of anionic polymer containing carboxyl groups and cationic polymer containing amino groups based on varenicline tartrate]

A film formulation containing varenicline tartrate as an active ingredient was prepared in the same manner as in Example 1, by varying the weight ratio of varenicline tartrate, an anionic polymer containing carboxyl groups and a cationic polymer containing amino groups as described in Table 4.
The upper weight ratio limit and the lower weight ratio limit of the anionic polymer containing carboxyl groups and the cationic polymer containing amino groups, which are bitterness masking agents, were set to 1:100:100 and 10:1:1, respectively.
The bitterness masking effect of the prepared film formulation was tested in the same manner as described in Test Example 1.
Tensile strength was tested by the following method.
After coating the solution for forming a film on a support film and cutting to a predetermined size, the formed film was detached from the support film and tensile strength was measured using a universal testing machine (texture analyzer) under the following condition.

| | |
|---|---|
| Universal testing machine | LLOYD LS-1 |
| Load cell (N) | 100N |
| Grips | Pneumatic vice grips |
| Testing speed (mm/min) | 200 |
| Distance between the grips (mm) | 2 |
| Air pressure of the grip (MPa) | 0 |

The tensile strength was calculated from the measurement results according to the following equation.

$$\text{Tensile strength} = \text{load value (N)} / \text{sample area (mm}^2\text{)}$$

Flexibility was evaluated as follows. After coating the solution for forming a film on a support film and cutting to a predetermined size, the formed film was detached from the support film. After folding the formed film, the maximum angle at which the film was not broken was measured.
The result is shown in Table 4.

[Table 4]

| | | Comparative Example | Example (%) | | | | |
|---|---|---|---|---|---|---|---|
| | | 1 | 2 | 3 | 4 | 5 | 6 |
| Ratio (API: Anionic polymer: Cationic polymer) | | - | 10:1:1 | 5:4:1 | 1:8:2 | 1:40:10 | 1:100:100 |
| Varenicline Tartrate | | 0.25 | 0.25 | 0.24 | 0.23 | 0.22 | 0.17 |
| Taste masking agent | Purolite C108DR | - | 0.02 | 0.19 | 1.87 | 8.36 | 17.00 |
| | Eudragit EPO | - | 0.02 | 0.05 | 0.47 | 2.21 | 17.00 |
| Pullulan | | 13.00 | | | | | |
| Plasticizer | | 7.00 | | | | | |
| Aspartame | | 3.00 | | | | | |
| Pigment | | 10.00 | | | | | |
| Diluent | | Q.S | | | | | |
| Flavor | | Q.S | | | | | |
| Water | | to 100% | | | | | |
| | | | | | | | |
| Evaluation parameter | Weigh | Example No. | | | | | |
| | | 1 | 2 | 3 | 4 | 5 | 6 |
| Effect of taste masking | 2 | 2 | 4 | 6 | 8 | 8 | 6 |
| Tensile strength | 1 | 4 | 4 | 4 | 4 | 3 | 2 |
| Flexibility | 1 | 4 | 4 | 4 | 4 | 3 | 2 |
| Total score | | 10 | 12 | 14 | 16 | 14 | 10 |

*** Taste masking value**

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |

*** Tensile strength**

| | |
|---|---|
| 1 - poor | Tensile strength value : NMT 5 $N/mm^2$ |
| 2 - not good | Tensile strength value : NMT 10 $N/mm^2$ |
| 3 - not bad | Tensile strength value : NLT 10 $N/mm^2$ |
| 4 - excellent | Tensile strength value : NLT 15 $N/mm^2$ |

*** Flexibility**

| | |
|---|---|
| 1 - poor | Maximised folding angle without breaking NMT 60° |
| 2 - not good | Maximised folding angle without breaking NMT 90° |

(continued)

| | |
|---|---|
| 3 - not bad | Maximised folding angle without breaking : NLT 90° |
| 4 - excellent | Maximsed folding angle without breaking : NLT 150° |
| (NLT: no less than, NMT: no more than) | |

As seen from Table 4, the most superior effect was achieved in terms of the bitterness masking effect, tensile strength and flexibility considered together when the weight ratio was 1:8:2.

[Test Example 5. Test of rapidly disintegrating tablet depending on weight ratio of anionic polymer containing carboxyl groups and cationic polymer containing amino groups based on varenicline tartrate]

A rapidly disintegrating tablet containing varenicline tartrate as an active ingredient was prepared in the same manner as in Example 3, by varying the weight ratio of varenicline tartrate, an anionic polymer containing carboxyl groups and a cationic polymer containing amino groups as described in Table 5.
The upper weight ratio limit and the lower weight ratio limit of the anionic polymer containing carboxyl groups and the cationic polymer containing amino groups, which are bitterness masking agents, were set to 1:100:100 and 10:1:1, respectively.
The bitterness masking effect of the prepared rapidly disintegrating tablet was tested in the same manner as described in Test Example 1.
Disintegration time was tested according to the Korean Pharmacopoeia General Test Method.
The result is shown in Table 5.

[Table 5]

| | | Comparative Example | Example (%) | | | | |
|---|---|---|---|---|---|---|---|
| | | 1 | 2 | 3 | 4 | 5 | 6 |
| Ratio (API : Anionic polymer: Cationic polymer) | | - | 10:1:1 | 5:4:1 | 1:8:2 | 1:40:10 | 1:100:100 |
| Varenicline Tartrate | | 0.25 | 0.25 | 0.24 | 0.23 | 0.22 | 0.17 |
| Taste masking agent | Purolite C108DR | - | 0.02 | 0.19 | 1.87 | 8.36 | 17.00 |
| | Eudragit EPO | - | 0.02 | 0.05 | 0.47 | 2.21 | 17.00 |
| Binder & Disintegration agent & Diluent | | 60.00 | | | | | |
| Aspartame | | 7.00 | | | | | |
| Flavor (If needed) | | Q.S | | | | | |
| Lubricant (If needed) | | Q.S | | | | | |
| Water | | to 100% | | | | | |
| | | | | | | | |
| Evaluation parameter | Weigh | Example No. | | | | | |
| | | 1 | 2 | 3 | 4 | 5 | 6 |
| Effect of taste masking | 2 | 2 | 4 | 6 | 8 | 8 | 6 |
| Distintegration | 1 | 4 | 4 | 4 | 4 | 3 | 2 |
| Total score | | 6 | 8 | 10 | 12 | 11 | 8 |

*** Taste masking value**

| | |
|---|---|
| 1 - poor | Retention time of Bitter, burning effects : NLT 60min. |

(continued)

| | |
|---|---|
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |

*** Disintergration time**

| | |
|---|---|
| 1 - poor | Retention time of ODT in sinker : NLT 2min |
| 2 - not good | Retention time of ODT in sinker : NLT 1min |
| 3 - not bad | Retention time of ODT in sinker : NMT 1min |
| 4 - excellent | Retention time of ODT in sinker : NMT 30sec |

*The test is performed using disintegration tester
(NLT: no less than, NMT: no more than)

As seen from Table 5, the most superior effect was achieved in terms of the bitterness masking effect and disintegration time considered together when the weight ratio was 1:8:2.

[Test Example 6. Test of bitterness masking effect of film formulation containing varenicline salicylate as active ingredient depending on weight ratio of anionic polymer containing carboxyl groups and cationic polymer containing amino groups]

A film formulation containing varenicline salicylate as an active ingredient was prepared in the same manner as in Example 2, using a combination of an anionic polymer containing carboxyl groups and a cationic polymer containing amino groups as described in Table 6 by varying their weight ratio.
The bitterness masking effect of the film formulation was tested in the same manner as described in Test Example 1.
The result is shown in Table 6.

[Table 6]

| | | Comparative Example | Example % | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| Ratio (API: TM agent) | | - | 10:1 | | | | | 1:10 | | | | | 1:100 | | | | |
| Ratio of TM agent (Anionic polymer: Cationic polymer) | | | 1:10 | 5:1 | 4:1 | 1:4 | 20:1 | 1:10 | 5:1 | 4:1 | 1:4 | 20:1 | 1:10 | 5:1 | 4:1 | 1:4 | 20:1 |
| Varenicline Salicylate | | 0.26 | 0.26 | | | | | 0.25 | | | | | 0.21 | | | | |
| Taste masking agent | Purolite C108DR | - | 0.002 | 0.019 | 0.019 | 0.005 | 0.019 | 0.210 | 1.941 | 1.867 | 0.467 | 2.220 | 1.751 | 16.049 | 15.426 | 3.857 | 18.359 |
| | Eudragit EPO | - | 0.021 | 0.004 | 0.005 | 0.019 | 0.001 | 2.100 | 0.387 | 0.467 | 1.867 | 0.107 | 17.513 | 3.202 | 3.857 | 15.426 | 0.918 |
| Pullulan | | 13.0 | | | | | | | | | | | | | | | |
| Plasticizer | | 7.0 | | | | | | | | | | | | | | | |
| Aspartame | | 3.0 | | | | | | | | | | | | | | | |
| Pigment | | 10.0 | | | | | | | | | | | | | | | |
| Diluent | | Q.S | | | | | | | | | | | | | | | |
| Flavor | | Q.S | | | | | | | | | | | | | | | |
| Water | | to 100% | | | | | | | | | | | | | | | |
| Effect of taste masking | | 1 | 2 | 3 | 3 | 3 | 2 | 3 | 4 | 4 | 4 | 3 | 3 | 4 | 4 | 4 | 3 |

* Taste masking value

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |
| (NLT: no less than, NMT: no more than) | |

As seen from Table 6, superior bitterness masking effect was achieved for the varenicline salicylate (API) when the weight ratio of the anionic polymer containing carboxyl groups and the cationic polymer containing amino groups used as the bitter taste masking agents was in the range from 5:1 to 1:4.

[Test Example 7. Test of bitterness masking effect of rapidly disintegrating tablet containing varenicline salicylate as active ingredient depending on weight ratio of anionic polymer containing carboxyl groups and cationic polymer containing amino groups]

A rapidly disintegrating tablet containing varenicline salicylate as an active ingredient was prepared in the same manner as in Example 2, using a combination of an anionic polymer containing carboxyl groups and a cationic polymer containing amino groups as described in Table 7 by varying their weight ratio.

The bitterness masking effect of the film formulation was tested in the same manner as described in Test Example 1.

The result is shown in Table 7.

[Table 7]

| | | Comparative Example | Example % | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| Ratio (API: TM agent) | | | 10:1 | | | | | 1:10 | | | | | 1:100 | | | | |
| Ratio of TM agent (Anionic polymer: Cationic polymer) | | | 1:10 | 5:1 | 4:1 | 1:4 | 20:1 | 1:10 | 5:1 | 4:1 | 1:4 | 20:1 | 1:10 | 5:1 | 4:1 | 1:4 | 20:1 |
| Varenicline Salicylate | | 0.26 | 0.26 | | | | | 0.25 | | | | | 0.21 | | | | |
| Taste masking agent | Purolite C108DR | - | 0.002 | 0.019 | 0.019 | 0.005 | 0.019 | 0.210 | 1.941 | 1.867 | 0.467 | 2.220 | 1.751 | 16.049 | 15.426 | 3.857 | 18.359 |
| | Eudragit EPO | - | 0.021 | 0.004 | 0.005 | 0.019 | 0.001 | 2.100 | 0.387 | 0.467 | 1.867 | 0.107 | 17.513 | 3.202 | 3.857 | 15.426 | 0.918 |
| Binder & Disintegration agent & Diluent | | 60.0 | | | | | | | | | | | | | | | |
| Aspartame | | 7.0 | | | | | | | | | | | | | | | |
| Flavor (If needed) | | Q.S | | | | | | | | | | | | | | | |
| Lubricant (If needed) | | Q.S | | | | | | | | | | | | | | | |
| Water | | to 100% | | | | | | | | | | | | | | | |
| Effect of taste masking | | 1 | 2 | 3 | 3 | 3 | 2 | 3 | 4 | 4 | 4 | 3 | 3 | 4 | 4 | 4 | 3 |

* Taste masking value

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |
| (NLT: no less than, NMT: no more than) | |

As seen from Table 7, superior bitterness masking effect was achieved for the varenicline salicylate (API) when the weight ratio of the anionic polymer containing carboxyl groups and the cationic polymer containing amino groups used as the bitter taste masking agents was in the range from 5:1 to 1:4.

[Test Example 8. Test of film formulation depending on weight ratio of anionic polymer containing carboxyl groups and cationic polymer containing amino groups based on varenicline salicylate]

A film formulation containing varenicline salicylate as an active ingredient was prepared in the same manner as in Example 2, by varying the weight ratio of varenicline salicylate, an anionic polymer containing carboxyl groups and a cationic polymer containing amino groups as described in Table 8.

The upper weight ratio limit and the lower weight ratio limit of the anionic polymer containing carboxyl groups and the cationic polymer containing amino groups, which are bitterness masking agents, were set to 1:100:100 and 10:1:1, respectively.

The bitterness masking effect of the prepared film formulation was tested in the same manner as described in Test Example 1.

Tensile strength was tested by the following method.

After coating the solution for forming a film on a support film and cutting to a predetermined size, the formed film was detached from the support film and tensile strength was measured using a universal testing machine (texture analyzer) under the following condition.

| | |
|---|---|
| Universal testing machine | LLOYD LS-1 |
| Load cell (N) | 100N |
| Grips | Pneumatic vice grips |
| Testing speed (mm/min) | 200 |
| Distance between the grips (mm) | 2 |
| Air pressure of the grip (MPa) | 0 |

The tensile strength was calculated from the measurement results according to the following equation.

$$\text{Tensile strength} = \text{load value (N)} / \text{sample area (mm}^2\text{)}$$

Flexibility was evaluated as follows. After coating the solution for forming a film on a support film and cutting to a predetermined size, the formed film was detached from the support film. After folding the formed film, the maximum angle at which the film was not broken was measured.

The result is shown in Table 8.

[Table 8]

| | Comparative Example | Example (%) | | | | |
|---|---|---|---|---|---|---|
| | 1 | 2 | 3 | 4 | 5 | 6 |
| Ratio (API: Anionic polymer: Cationic polymer) | - | 10:1:1 | 5:4:1 | 1:8:2 | 1:40:10 | 1:100:100 |
| Varenicline Salicylate | 0.26 | 0.27 | 0.26 | 0.25 | 0.24 | 0.19 |

(continued)

| | | Comparative Example | Example (%) | | | | |
|---|---|---|---|---|---|---|---|
| | | 1 | 2 | 3 | 4 | 5 | 6 |
| Taste masking agent | Purolite C108DR | - | 0.02 | 0.19 | 1.87 | 8.36 | 17.00 |
| | Eudragit EPO | - | 0.02 | 0.05 | 0.47 | 2.21 | 17.00 |
| Pullulan | | 13.00 | | | | | |
| Plasticizer | | 7.00 | | | | | |
| Aspartame | | 3.00 | | | | | |
| Pigment | | 10.00 | | | | | |
| Diluent | | Q.S | | | | | |
| Flavor | | Q.S | | | | | |
| Water | | to 100% | | | | | |

| Evaluation parameter | Weigh | Example No. | | | | | |
|---|---|---|---|---|---|---|---|
| | | 1 | 2 | 3 | 4 | 5 | 6 |
| Effect of taste masking | 2 | 2 | 4 | 6 | 8 | 8 | 8 |
| Tensile strength | 1 | 4 | 4 | 4 | 4 | 3 | 2 |
| Flexibility | 1 | 4 | 4 | 4 | 4 | 3 | 2 |
| Total score | | 10 | 12 | 14 | 16 | 14 | 12 |

*** Taste masking value**

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |

*** Tensile strength**

| | |
|---|---|
| 1 - poor | Tensile strength value : NMT 5 $N/mm^2$ |
| 2 - not good | Tensile strength value : NMT 10 $N/mm^2$ |
| 3 - not bad | Tensile strength value : NLT 10 $N/mm^2$ |
| 4 - excellent | Tensile strength value : NLT 15 $N/mm^2$ |

*** Flexibility**

| | |
|---|---|
| 1 - poor | Maximised folding angle without breaking NMT 60° |
| 2 - not good | Maximised folding angle without breaking NMT 90° |
| 3 - not bad | Maximised folding angle without breaking : NLT 90° |
| 4 - excellent | Maximsed folding angle without breaking : NLT 150° |

(NLT: no less than, NMT: no more than)

As seen from Table 8, the most superior effect was achieved in terms of the bitterness masking effect, tensile strength and flexibility considered together when the weight ratio was 1:8:2.

[Test Example 9. Test of rapidly disintegrating tablet depending on weight ratio of anionic polymer containing carboxyl groups and cationic polymer containing amino groups based on varenicline salicylate]

A rapidly disintegrating tablet containing varenicline salicylate as an active ingredient was prepared in the same manner as in Example 4, by varying the weight ratio of varenicline salicylate, an anionic polymer containing carboxyl groups and a cationic polymer containing amino groups as described in Table 9.
The upper weight ratio limit and the lower weight ratio limit of the anionic polymer containing carboxyl groups and the cationic polymer containing amino groups, which are bitterness masking agents, were set to 1:100:100 and 10:1:1, respectively.
The bitterness masking effect of the prepared rapidly disintegrating tablet was tested in the same manner as described in Test Example 1.
Disintegration time was tested according to the Korean Pharmacopoeia General Test Method.
The result is shown in Table 9.

[Table 9]

| | | | Comparative Example | Example (%) | | | | |
|---|---|---|---|---|---|---|---|---|
| | | | 1 | 2 | 3 | 4 | 5 | 6 |
| Ratio (API: Anionic polymer: Cationic polymer) | | | - | 10:1:1 | 5:4:1 | 1:8:2 | 1:40:10 | 1:100:100 |
| Varenicline Salicylate | | | 0.27 | 0.27 | 0.26 | 0.25 | 0.24 | 0.19 |
| Taste masking agent | Purolite C108DR | | - | 0.02 | 0.19 | 1.87 | 8.36 | 17.00 |
| | Eudragit EPO | | - | 0.02 | 0.05 | 0.47 | 2.21 | 17.00 |
| Binder & Disintegration agent & Diluent | | | 60.00 | | | | | |
| Aspartame | | | 7.00 | | | | | |
| Flavor (If needed) | | | Q.S | | | | | |
| Lubricant (If needed) | | | Q.S | | | | | |
| Water | | | to 100% | | | | | |
| | | | | | | | | |
| Evaluation parameter | | Weigh | Example No. | | | | | |
| | | | 1 | 2 | 3 | 4 | 5 | 6 |
| Effect of taste masking | | 2 | 2 | 4 | 6 | 8 | 8 | 8 |
| Distintegration | | 1 | 4 | 4 | 4 | 4 | 3 | 2 |
| Total score | | | 6 | 8 | 10 | 12 | 11 | 10 |

*** Taste masking value**

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |

*** Disintergration time**

| | |
|---|---|
| 1 - poor | Retention time of ODT in sinker: NLT 2min |
| 2 - not good | Retention time of ODT in sinker: NLT 1min |
| 3 - not bad | Retention time of ODT in sinker: NMT 1min |
| 4 - excellent | Retention time of ODT in sinker: NMT 30sec |

*The test is performed using disintegration tester
(NLT: no less than, NMT: no more than)

As seen from Table 9, the most superior effect was achieved in terms of the bitterness masking effect and disintegration time considered together when the weight ratio was 1:8:2.

[Test Example 10. Test of difference in bitterness masking effect of film formulation depending on change in varenicline salts]

A film formulation was prepared in the same manner as in Example 1 and Example 2, by varying the varenicline salt as described in Table 10 and using an anionic polymer containing carboxyl groups and a cationic polymer containing amino groups as bitterness masking agents.
The bitterness masking effect of the prepared film formulation was tested in the same manner as described in Test Example 1.
The result is shown in Table 10.

[Table 10]

| | | Comparative Example (%) | | | | | | | | | | | | | | Example (%) | | | | | | | | | | | | | |
|---|---|---|---|---|---|---|---|---|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 1 | 2 | 3 | 4 | 5 | 6 | 7 | 8 | 9 | 10 | 11 | 12 | 13 | 14 |
| Varenicline Tartrate | | 0.23 | - | - | - | - | - | - | - | - | - | - | - | - | - | 0.23 | - | - | - | - | - | - | - | - | - | - | - | - | - |
| Varenicline Salicylate | | - | 0.23 | - | - | - | - | - | - | - | - | - | - | - | - | - | 0.23 | - | - | - | - | - | - | - | - | - | - | - | - |
| Varenicline Sulfate | | - | - | 0.23 | - | - | - | - | - | - | - | - | - | - | - | - | - | 0.23 | - | - | - | - | - | - | - | - | - | - | - |
| Varenicline Fumarate | | - | - | - | 0.23 | - | - | - | - | - | - | - | - | - | - | - | - | - | 0.23 | - | - | - | - | - | - | - | - | - | - |
| Varenicline Oxalate | | - | - | - | - | 0.23 | - | - | - | - | - | - | - | - | - | - | - | - | - | 0.23 | - | - | - | - | - | - | - | - | - |
| Varenicline Hydrochloride | | - | - | - | - | - | 0.23 | - | - | - | - | - | - | - | - | - | - | - | - | - | 0.23 | - | - | - | - | - | - | - | - |
| Varenicline Hydrobromide | | - | - | - | - | - | - | 0.23 | - | - | - | - | - | - | - | - | - | - | - | - | - | 0.23 | - | - | - | - | - | - | - |
| Varenicline Citrate | | - | - | - | - | - | - | - | 0.23 | - | - | - | - | - | - | - | - | - | - | - | - | - | 0.23 | - | - | - | - | - | - |
| Varenicline Malate | | - | - | - | - | - | - | - | - | 0.23 | - | - | - | - | - | - | - | - | - | - | - | - | - | 0.23 | - | - | - | - | - |
| Varenicline Succinate | | - | - | - | - | - | - | - | - | - | 0.23 | - | - | - | - | - | - | - | - | - | - | - | - | - | 0.23 | - | - | - | - |
| Varenicline Phosphate | | - | - | - | - | - | - | - | - | - | - | 0.23 | - | - | - | - | - | - | - | - | - | - | - | - | - | 0.23 | - | - | - |
| Varenicline Tosylate | | - | - | - | - | - | - | - | - | - | - | - | 0.23 | - | - | - | - | - | - | - | - | - | - | - | - | - | 0.23 | - | - |
| Varenicline Free Base | | - | - | - | - | - | - | - | - | - | - | - | - | 0.23 | - | - | - | - | - | - | - | - | - | - | - | - | - | 0.23 | - |
| Varenicline Mesylate | | - | - | - | - | - | - | - | - | - | - | - | - | - | 0.23 | - | - | - | - | - | - | - | - | - | - | - | - | - | 0.23 |
| TM agent | Purolite C108DR | - | | | | | | | | | | | | | | 1.87 | | | | | | | | | | | | | |
| | Eudragit EPO | - | | | | | | | | | | | | | | 0.47 | | | | | | | | | | | | | |
| Pullulan | | 13 | | | | | | | | | | | | | | | | | | | | | | | | | | | |
| Plasticizer | | 7 | | | | | | | | | | | | | | | | | | | | | | | | | | | |
| Aspartame | | 3 | | | | | | | | | | | | | | | | | | | | | | | | | | | |
| Pigment | | 10 | | | | | | | | | | | | | | | | | | | | | | | | | | | |
| Diluent | | Q.S | | | | | | | | | | | | | | | | | | | | | | | | | | | |
| Flavor | | Q.S | | | | | | | | | | | | | | | | | | | | | | | | | | | |
| Water | | up to 100% | | | | | | | | | | | | | | | | | | | | | | | | | | | |
| Effect of taste masking | | 1 | 1 | 1 | 1 | 1 | 1 | 1 | 1 | 1 | 1 | 1 | 1 | 1 | 1 | 4 | 4 | 4 | 4 | 4 | 3 | 3 | 4 | 4 | 4 | 3 | 4 | 3 | 4 |

* Taste masking value

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |
| (NLT: no less than, NMT: no more than) | |

As a result, superior bitterness masking effect was achieved for all of varenicline tartrate, varenicline salicylate, varenicline sulfate, varenicline fumarate, varenicline oxalate, varenicline hydrochloride, varenicline hydrobromide, varenicline citrate, varenicline maleate, varenicline succinate, varenicline phosphate, varenicline tosylate, varenicline free base and varenicline mesylate, when the anionic polymer containing carboxyl groups and the cationic polymer containing amino groups were used as bitterness masking agents.

[Test Example 11. Test of difference in bitterness masking effect of rapidly disintegrating tablet depending on change in varenicline salts]

A rapidly disintegrating tablet was prepared in the same manner as in Example 3 and Example 4, by varying the varenicline salt as described in Table 11 and using an anionic polymer containing carboxyl groups and a cationic polymer containing amino groups as bitterness masking agents.
The bitterness masking effect of the prepared rapidly disintegrating tablet was tested in the same manner as described in Test Example 1.
The result is shown in Table 11.

[Table 11]

| | | Comparative Example (%) | | | | | | | | | | | | | | Example (%) | | | | | | | | | | | | | |
|---|---|---|---|---|---|---|---|---|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 1 | 2 | 3 | 4 | 5 | 6 | 7 | 8 | 9 | 10 | 11 | 12 | 13 | 14 |
| Varenicline Tartrate | | 0.23 | - | - | - | - | - | - | - | - | - | - | - | - | - | 0.23 | - | - | - | - | - | - | - | - | - | - | - | - | - |
| Varenicline Salicylate | | - | 0.23 | - | - | - | - | - | - | - | - | - | - | - | - | - | 0.23 | - | - | - | - | - | - | - | - | - | - | - | - |
| Varenicline Sulfate | | - | - | 0.23 | - | - | - | - | - | - | - | - | - | - | - | - | - | 0.23 | - | - | - | - | - | - | - | - | - | - | - |
| Varenicline Fumarate | | - | - | - | 0.23 | - | - | - | - | - | - | - | - | - | - | - | - | - | 0.23 | - | - | - | - | - | - | - | - | - | - |
| Varenicline Oxalate | | - | - | - | - | 0.23 | - | - | - | - | - | - | - | - | - | - | - | - | - | 0.23 | - | - | - | - | - | - | - | - | - |
| Varenicline Hydrochloride | | - | - | - | - | - | 0.23 | - | - | - | - | - | - | - | - | - | - | - | - | - | 0.23 | - | - | - | - | - | - | - | - |
| Varenicline Hydrobromide | | - | - | - | - | - | - | 0.23 | - | - | - | - | - | - | - | - | - | - | - | - | - | 0.23 | - | - | - | - | - | - | - |
| Varenicline Citrate | | - | - | - | - | - | - | - | 0.23 | - | - | - | - | - | - | - | - | - | - | - | - | - | 0.23 | - | - | - | - | - | - |
| Varenicline Malate | | - | - | - | - | - | - | - | - | 0.23 | - | - | - | - | - | - | - | - | - | - | - | - | - | 0.23 | - | - | - | - | - |
| Varenicline Succinate | | - | - | - | - | - | - | - | - | - | 0.23 | - | - | - | - | - | - | - | - | - | - | - | - | - | 0.23 | - | - | - | - |
| Varenicline Phosphate | | - | - | - | - | - | - | - | - | - | - | 0.23 | - | - | - | - | - | - | - | - | - | - | - | - | - | 0.23 | - | - | - |
| Varenicline Tosylate | | - | - | - | - | - | - | - | - | - | - | - | 0.23 | - | - | - | - | - | - | - | - | - | - | - | - | - | 0.23 | - | - |
| Varenicline Free Base | | - | - | - | - | - | - | - | - | - | - | - | - | 0.23 | - | - | - | - | - | - | - | - | - | - | - | - | - | 0.23 | - |
| Varenicline Mesylate | | - | - | - | - | - | - | - | - | - | - | - | - | - | 0.23 | - | - | - | - | - | - | - | - | - | - | - | - | - | 0.23 |
| TM agent | Purolite C108DR | - | | | | | | | | | | | | | | 1.87 | | | | | | | | | | | | | |
| | Eudragit EPO | - | | | | | | | | | | | | | | 0.47 | | | | | | | | | | | | | |
| Binder & Disintegration agent & Diluent | | 60 | | | | | | | | | | | | | | | | | | | | | | | | | | | |
| Aspartame | | 7 | | | | | | | | | | | | | | | | | | | | | | | | | | | |
| Flavor (If needed) | | Q.S | | | | | | | | | | | | | | | | | | | | | | | | | | | |
| Lubricant (If needed) | | Q.S | | | | | | | | | | | | | | | | | | | | | | | | | | | |
| Water | | up to 100% | | | | | | | | | | | | | | | | | | | | | | | | | | | |
| Effect of taste masking | | 1 | 1 | 1 | 1 | 1 | 1 | 1 | 1 | 1 | 1 | 1 | 1 | 1 | 1 | 4 | 4 | 4 | 4 | 4 | 3 | 3 | 4 | 4 | 4 | 3 | 4 | 3 | 4 |

* Taste masking value

| | |
|---|---|
| 1 - poor | Retention time of Bitter, burning effects : NLT 60min. |
| 2 - not good | Retention time of Bitter, burning effects : NLT 20min. |
| 3 - not bad | Retention time of Bitter, burning effects : NMT 20min. |
| 4 - excellent | Retention time of Bitter, burning effects : NMT 5min. |
| (NLT: no less than, NMT: no more than) | |

As a result, superior bitterness masking effect was achieved for all of varenicline tartrate, varenicline salicylate, varenicline sulfate, varenicline fumarate, varenicline oxalate, varenicline hydrochloride, varenicline hydrobromide, varenicline citrate, varenicline maleate, varenicline succinate, varenicline phosphate, varenicline tosylate, varenicline free base and varenicline mesylate, when the anionic polymer containing carboxyl groups and the cationic polymer containing amino groups were used as bitterness masking agents.

## Claims

1. A bitterness-masked orally administered pharmaceutical preparation comprising varenicline or a pharmaceutically acceptable salt thereof as an active ingredient and comprising both an anionic polymer comprising carboxyl groups and a cationic polymer comprising amino groups.

2. The bitterness-masked orally administered pharmaceutical preparation according to claim 1, wherein the active ingredient is one or more selected from a group consisting of varenicline tartrate, varenicline salicylate, varenicline sulfate, varenicline fumarate, varenicline oxalate, varenicline hydrochloride, varenicline hydrobromide, varenicline citrate, varenicline maleate, varenicline succinate, varenicline phosphate, varenicline tosylate, varenicline free base and varenicline mesylate.

3. The bitterness-masked orally administered pharmaceutical preparation according to claim 1, wherein the anionic polymer comprising carboxyl groups is a polyacryl-based anionic polymer, a polystyrene-based anionic polymer or a mixture thereof.

4. The bitterness-masked orally administered pharmaceutical preparation according to claim 1, wherein the anionic polymer comprising carboxyl groups is one or more of a crosslinked polymer of methacrylic acid and divinylbenzene, a crosslinked polymer of styrene and divinylbenzene, a crosslinked polymer of acrylic acid and divinylbenzene or a mixture thereof.

5. The bitterness-masked orally administered pharmaceutical preparation according to claim 1, wherein the cationic polymer comprising amino groups is a copolymer polymerized from methacrylic acid, acrylic acid or styrene as a monomer and comprising amino groups.

6. The bitterness-masked orally administered pharmaceutical preparation according to claim 1, wherein the weight ratio of the total weight of the cationic polymer comprising amino groups based on the total weight of the anionic polymer comprising carboxyl groups is 5:1 to 1:4.

7. The bitterness-masked orally administered pharmaceutical preparation according to claim 1, wherein the weight ratio of the total weight of the anionic polymer comprising carboxyl groups and the cationic polymer comprising amino groups based on the total weight of the varenicline or a pharmaceutically acceptable salt thereof is 10:1 to 1:100.

8. The bitterness-masked orally administered pharmaceutical preparation according to claim 1, wherein the weight ratio of the total weight of the varenicline or a pharmaceutically acceptable salt thereof, the total weight of the anionic polymer comprising carboxyl groups and the total weight of the cationic polymer comprising amino groups is 1:8:2.

9. The bitterness-masked orally administered pharmaceutical preparation according to claim 1, wherein the orally administered pharmaceutical preparation is a smoking cessation aid.

10. The bitterness-masked orally administered pharmaceutical preparation according to claim 1, wherein the orally administered pharmaceutical preparation is dissolved or disintegrated in the mouth.

**11.** The bitterness-masked orally administered pharmaceutical preparation according to claim 10, wherein the orally administered pharmaceutical preparation is a rapidly disintegrating tablet or a film formulation.

## INTERNATIONAL SEARCH REPORT

International application No.
PCT/KR2017/000290

A. CLASSIFICATION OF SUBJECT MATTER

*A61K 47/30(2006.01)i, A61K 9/00(2006.01)i, A61K 31/498(2006.01)i, A61K 31/4985(2006.01)i*

According to International Patent Classification (IPC) or to both national classification and IPC

B. FIELDS SEARCHED

Minimum documentation searched (classification system followed by classification symbols)
A61K 47/30; A61K 9/14; A61K 9/22; A61K 47/36; A61K 31/498; A61K 9/20; A61K 47/38; A61K 9/28; A61K 9/00; A61K 31/4985

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: Varenicline, anion polymer, carboxyl group, cationic polymer, amino acid, taste masking

C. DOCUMENTS CONSIDERED TO BE RELEVANT

| Category* | Citation of document, with indication, where appropriate, of the relevant passages | Relevant to claim No. |
|---|---|---|
| A | US 2006-0057207 A1 (ZIEGLER, C. B. et al.) 16 March 2006<br>See abstract and claims 1-18. | 1-11 |
| A | US 2006-0084656 A1 (ZIEGLER, C. B. et al.) 20 April 2006<br>See the entire document. | 1-11 |
| A | KR 10-2010-0108307 A (MCNEIL AB.) 06 October 2010<br>See the entire document. | 1-11 |
| A | US 2014-0018404 A1 (CHEN, M. J. et al.) 16 January 2014<br>See the entire document. | 1-11 |
| A | WO 2009-034431 A2 (PFIZER INC.) 19 March 2009<br>See the entire document. | 1-11 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 APRIL 2017 (26.04.2017) | **26 APRIL 2017 (26.04.2017)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2017/000290**

| Patent document cited in search report | Publication date | Patent family member | Publication date |
|---|---|---|---|
| US 2006-0057207 A1 | 16/03/2006 | AU 2002-351008 A1 | 10/06/2003 |
| | | AU 2002-351008 B2 | 12/07/2007 |
| | | CA 2467490 A1 | 05/06/2003 |
| | | CA 2467490 C | 09/01/2007 |
| | | CA 2563052 A1 | 05/06/2003 |
| | | CN 1864663 A | 22/11/2006 |
| | | CN 1864663 C | 22/11/2006 |
| | | EP 1448235 A1 | 25/08/2004 |
| | | EP 1448235 B1 | 14/03/2007 |
| | | JP 2005-528330 A | 22/09/2005 |
| | | JP 2009-108064 A | 21/05/2009 |
| | | KR 10-0892517 B1 | 10/04/2009 |
| | | KR 10-2004-0063975 A | 15/07/2004 |
| | | US 2003-0180360 A1 | 25/09/2003 |
| | | US 7771748 B2 | 10/08/2010 |
| | | WO 03-045437 A1 | 05/06/2003 |
| US 2006-0084656 A1 | 20/04/2006 | CA 2583101 A1 | 20/04/2006 |
| | | EP 1802276 A1 | 04/07/2007 |
| | | JP 2008-516942 A | 22/05/2008 |
| | | MX 2007004495 A | 10/05/2007 |
| | | WO 2006-040680 A1 | 20/04/2006 |
| KR 10-2010-0108307 A | 06/10/2010 | AU 2010-201229 A1 | 11/11/2010 |
| | | CA 2697867 A1 | 27/09/2010 |
| | | CN 101843581 A | 29/09/2010 |
| | | EP 2233134 A1 | 29/09/2010 |
| | | JP 2010-229131 A | 14/10/2010 |
| | | US 2010-0247586 A1 | 30/09/2010 |
| US 2014-0018404 A1 | 16/01/2014 | CA 2821805 A1 | 21/06/2012 |
| | | CN 103442698 A | 11/12/2013 |
| | | CN 103442698 B | 05/10/2016 |
| | | EP 2651400 A2 | 23/10/2013 |
| | | JP 2013-545814 A | 26/12/2013 |
| | | MX 2013006591 A | 04/11/2013 |
| | | WO 2012-083017 A2 | 21/06/2012 |
| | | WO 2012-083017 A3 | 23/08/2012 |
| WO 2009-034431 A2 | 19/03/2009 | WO 2009-034431 A3 | 19/11/2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020160002797
- WO 199935131 A
- WO 9935131 A
- US 6410550 B
- WO 0162736 A